# EUROPÄISCHE PATENTSCHRIFT

(11) **EP 1 194 088 B1**
(45) Veröffentlichungstag und Bekanntmachung des Hinweises auf die Patenterteilung: **12.03.2008**
(21) Anmeldenummer: 99931246.5
(22) Anmeldetag: 02.07.1999
(51) Int. Cl.: A61F 2/44

(54) **ZWISCHENWIRBELIMPLANTAT**
INTERVERTEBRAL IMPLANT
IMPLANT INTERVERTEBRAL

(43) Veröffentlichungstag der Anmeldung: 10.04.2002
(60) Teilanmeldung: 07004657.8
(73) Patentinhaber: Spine Solutions Inc., West Chester, PA 19380 (US)
(72) Erfinder: MARNAY, Thierry, F-34080 Montpellier (FR); BEYERSDORFF, Boris, D-10119 Berlin (DE)
(74) Vertreter: Erb, Henning
(86) Internationale Anmeldenummer: PCT/EP1999/004628
(87) Internationale Veröffentlichungsnummer: WO 2001/001893

(56) Entgegenhaltungen:
- EP-A- 0 333 990
- WO-A-98/14142
- FR-A- 2 718 635
- US-A- 5 314 477

## Beschreibung

Die Erfindung betrifft ein Zwischenwirbelimplantat gemäß dem Oberbegriff des beigefügten Anspruches 1.

Ein derartiges Zwischenwirbelimplantat ist beispielsweise aus der US-A-5,314,477 oder auch der US-A-5,507,816 bekannt. Dieses Zwischenwirbelimplantat dient dem Ersetzen einer aus dem Zwischenwirbelraum entfernten Bandscheibe, und dementsprechend muß das Zwischenwirbelimplantat eine relativ geringe Bauhöhe aufweisen, da es in den Zwischenwirbelspalt hineinpassen muß. Dies ist insbesondere dann schwierig, wenn zwischen dem Oberteil und dem Unterteil noch ein zusätzlicher Gelenkeinsatz eingebettet ist, wie dies bei dem bekannten Zwischenwirbelimplantat der US-A-5,314,477 der Fall ist.

Schwierigkeiten ergeben sich aber auch schon bei zweiteiligen Zwischenwirbelimplantaten insbesondere dann, wenn diese an ihren Stützflächen noch Stifte und andere Vorsprünge tragen, die der Verankerung des Zwischenwirbelimplantates im Knochen dienen sollen. Diese können dann häufig nur dadurch eingesetzt werden, daß der Zwischenwirbelraum stark aufgeweitet wird. Dies ist nicht nur sehr schwierig, sondern birgt auch die Gefahr von Verletzungen in sich.

Da der Zwischenwirbelraum eine relativ geringe Höhe aufweist, ist es auch schwierig, an den beiden Teilen des Zwischenwirbelimplantates Angriffselemente zu befestigen, an denen ein Handhabungsinstrument angreifen kann. Es ist üblich, derartige Handhabungsinstrumente getrennt am Oberteil und am Unterteil angreifen zu lassen, beispielsweise durch Stifte, die in Bohrungen am Oberteil bzw. am Unterteil eingesteckt werden, so daß mit dem Handhabungsinstrument die beiden Teile des Zwischenwirbelimplantates in den Zwischenwirbelraum eingesetzt und gegebenenfalls auch in ihrem Abstand voneinander verändert werden können, so daß dadurch eine gewisse Aufspreizung des Zwischenwirbelraumes möglich ist. Hierzu wird beispielsweise verwiesen auf das zangenförmige Handhabungsinstrument in der US-A-5,314.477.

Aufgrund der großen Kräfte ist es notwendig, für die Angriffselemente eine gewisse Bauhöhe vorzusehen, beispielsweise müssen die Aufnahmebohrungen einen bestimmten Durchmesser aufweisen. Daraus ergibt sich eine minimale Bauhöhe für das Oberteil und für das Unterteil, und bei herkömmlichen Zwischenwirbelimplantaten addieren sich somit die Bauhöhen von Oberteil und Unterteil, so daß selbst beim unmittelbaren Aufeinanderliegen von Oberteil und Unterteil eine relativ große Bauhöhe des Zwischenwirbelimplantates unvermeidlich ist.

Es ist Aufgabe der Erfindung, ein gattungsgemäßes Zwischenwirbelimplantat so auszubilden, daß die minimale Bauhöhe herabgesetzt wird, um das Einführen des Zwischenwirbelimplantates in den Zwischenwirbelraum zu erleichtern.

Diese Aufgabe wird bei einem Zwischenwirbelimplantat der eingangs beschriebenen Art erfindungegemäß durch die Merkmale des kennzeichnenden Teils des beigefügten Anspruches 1 gelöst.

Bei einer solchen Ausgestaltung läßt sich eine minimale Bauhöhe der beiden aufeinanderliegenden Zwischenwirbelimplantatsteile erreichen, da Bereiche großer Bauhöhe sind als Vorsprünge ausgebildet, neben denen sich jeweils Rücksprünge befinden, in die die Vorsprünge des jeweils anderen Teils eintauchen können. Daraus ergibt sich, daß die Angriffselemente für die Handhabungsinstrumente nebeneinander liegen und sich zumindest teilweise überlappen können, so daß die Gesamtbauhöhe der aufeinanderliegenden Teile des Zwischenwlrbelimplantates gegenüber herkömmlichen Zwischenwirbelimplantaten deutlich herabgesetzt werden kann. Es ergibt sich somit eine verschachtelte Anordnung von Oberteil und Unterteil mit maximaler Ausnützung der zur Verfügung stehenden Materialhöhe.

Dabei ist es günstig, wenn die Angriffselemente Einstecköffnungen für stiftförmige Halteelemente eines Kandhabungsinstrumentes sind, diese Einstecköffnungen können aufgrund der beschriebenen Konstruktion einen relativ großen Durchmesser aufweisen und damit kräftige Haltestifte aufnehmen, und trotzdem ergibt sich eine relativ geringe Bauhöhe des Zwischenwirbelimplantates bei unmittelbar aufeinandergelegten Teilen.

Dabei ist es vorteilhaft, wenn sich die Einstecköffnungen im wesentlichen parallel zu den Stützflächen erstrecken, auch dadurch wird vermieden, daß die Bauhöhe der Zwischenwlrbelimplantatstelle vergrößert wird.

Bei einer bevorzugten Ausführungsform ist vorgesehen, dass das zweite Teil, insbesondere das Unterteil eine der unteren Stützfläche gegenüberliegende zentrale Vertiefung aufweist, die von einem U-förmigen Rand umgeben ist.

Dabei ist es vorteilhaft, wenn das erste Teil, insbesondere das Oberteil, einen im wesentlichen komplementär in die Vertiefung passenden zentralen Vorsprung trägt, es wird also das gesamte Volumen der Vertiefung für den Vorsprung ausgenutzt.

Es ist weiterhin vorteilhaft, wenn die Angriffselemente des Unterteils an den beiden Enden des U-förmigen Randes angeordnet sind, also außen liegen.

Die Angriffselemente des Oberteils können dagegen an dem zentralen Vorsprung des Oberteils angeordnet sein, liegen also gegenüber den Angriffselementen des Oberteils weiter innen.

Insbesondere können die Angriffselemente des Oberteils nahe der seitlichen Ränder des zentralen Vorsprungs angeordnet sein, so daß auch für das Oberteil der Abstand der Angriffselemente relativ groß gewählt werden kann, dadurch läßt sich das Oberteil ebenso wie das Unterteil gegen eine Verkippung zuverlässig sichern.

Bereits hier sei darauf hingewiesen, daß die Ausdrücke "Unterteil" und "Oberteil" nicht unbedingt etwas über die Einbaulage des Zwischenwirbelimplantates in der Wirbelsäule aussagen, das mit "Unterteil" bezeichnete Teil könnte in der Wirbelsäule tatsächlich auch oben liegen. Wesentlich ist lediglich, daß Oberteil und Unterteil das Zwischenwirbelimplantat auf einander gegenüberliegenden Seiten des Implantates begrenzen.

Besonders vorteilhaft ist es, wenn das Oberteil und/oder das Unterteil im wesentlichen plattenförmig ausgebildet sind, wobei diese Teile natürlich entsprechend der erfindungsgemäßen Ausgestaltung Vor- und Rücksprünge aufweisen, die dem jeweils anderen Teil zugewandt sind. Die plattenförmige Ausbildung führt aber insgesamt zu einer sehr geringen Bauhöhe des Zwischenwirbelimplantates.

Bei einer bevorzugten Ausführungsform weisen sowohl das Unterteil als auch das Oberteil je eine Aufnahme für einen Gelenkeinsatz auf. Dieser Gelenkeinsatz, der nach dem Einsetzen des Zwischenwirbelimplantates zwischen Oberteil und Unterteil plaziert wird, stützt Oberteil und Unterteil gegeneinander ab, er übernimmt beispielsweise eine federnde Funktion und führt außerdem zu einer gewissen verschwenkbarkeit der beiden Teile eines zwischenwirbelimplantates gegeneinander, so daß damit auch eine Verschwenkbarkeit der benachbarten Wirbelkörper erreichbar ist.

Insbesondere ist es vorteilhaft, wenn der Gelenkeinsatz mindestens eine kugelige Oberseite aufweist, die in die entsprechend kugelig gewölbte Vertiefung eingreift.

Günstig ist es, wenn die kugelige Aufnahme in einem zentralen Vorsprung des Oberteils angeordnet ist.

Es ist weiterhin vorteilhaft, wenn die zentrale Vertiefung des Unterteils die Aufnahme für den Gelenkeinsatz bildet.

Gemäß einer bevorzugten Ausführungsform der Erfindung ist vorgesehen, daß der Gelenkeinsatz von der Seite in die Aufnahme einschiebbar ist, die die Angriffselemente für ein Handhabungsinstrument trägt. Es handelt sich dabei um die Seite, von der Oberteil und Unterteil in den Zwischenwirbelraum eingeführt werden, und von dieser Seite her kann dann auch der Gelenkeinsatz zwischen die bereits eingesetzten Teile des zwischenwirbelimplantats eingeschoben werden.

Dabei ist es günstig, wenn der Gelenkeinsatz längs einer Führung in die Aufnahme einschiebbar ist.

Auch der Einsatz ist vorzugsweise im wesentlichen plattenförmig ausgebildet.

Eine besonders günstige Ausgestaltung ergibt sich, wenn der Einsatz die zentrale Aufnahme im wesentlichen vollständig ausfüllt und mit der kugeligen Stützfläche aus der Aufnahme hervorsteht.

Die nachfolgende Beschreibung bevorzugter Ausführungsformen der Erfindung dient im Zusammenhang mit der Zeichnung der näheren Erläuterung. Es zeigen:

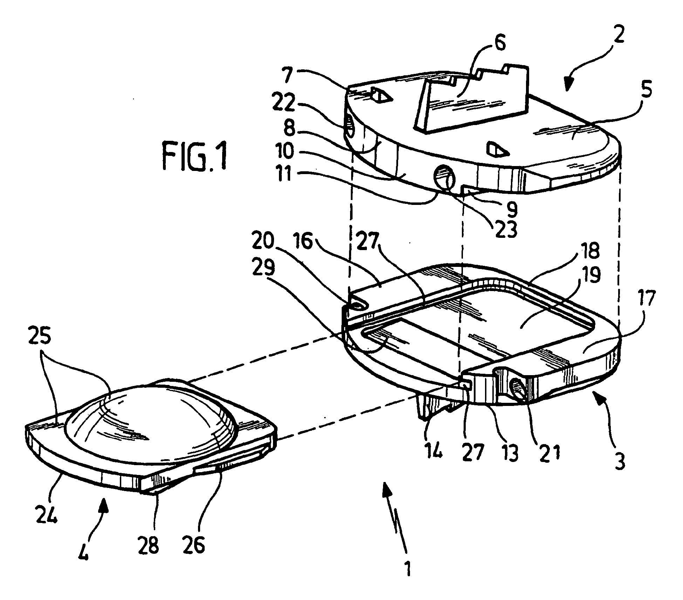

- Figur 1:: eine perspektivische Explosionsansicht eines Zwischenwirbelimplantates mit einem Oberteil, einem Unterteil und einem zwischen diese einsetzbaren Gelenkeinsatz;

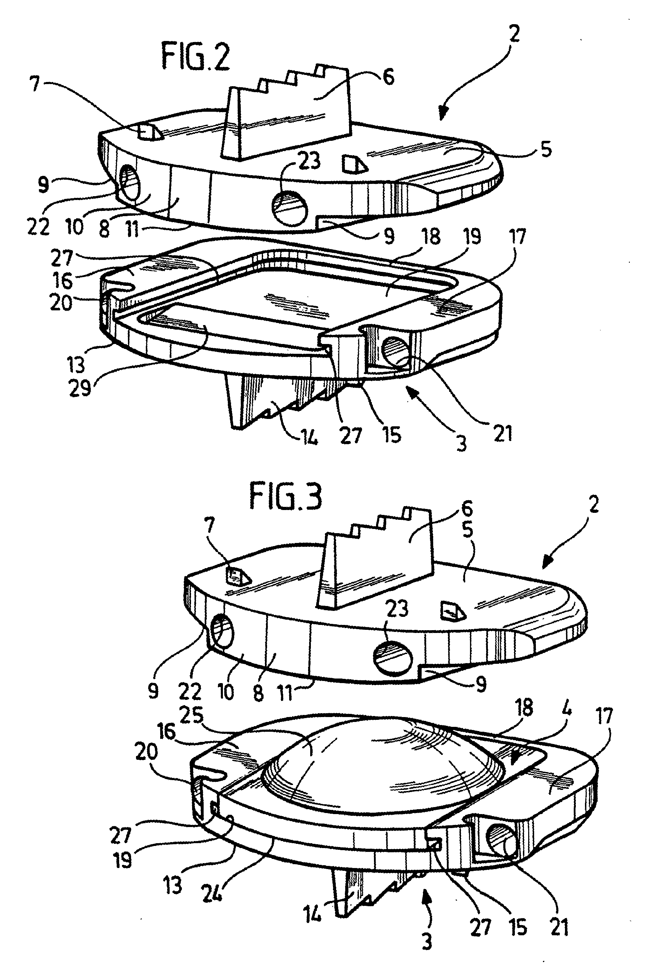

- Figur 2:: eine perspektivische Explosionsansicht des Oberteils und des Unterteils des Zwischenwirbelimplantates ohne eingesetzten Gelenkeinsatz;
- Figur 3:: eine Ansicht ähnlich Figur 2 mit in das Unterteil eingeschobenem Gelenkeinsatz;
- Figur 4:: eine perspektivische Ansicht des Oberteils und des Unterteils des Zwischenwirbelimplantates in maximaler gegenseitiger Annäherung;
- Figur 5:: eine Vorderansicht des Zwischenwirbelimplantats der Figur 4;
- Figur 6:: eine perspektivische Ansicht des Zwischenwirbelimplantates mit eingesetztem Gelenkeinsatz und
- Figur 7:: eine Querschnittsansicht des Zwischenwirbelimplantats der Figur 6.

Das in der Zeichnung dargestellte Zwischenwirbelimplantat 1 umfaßt drei Teile, nämlich ein plattenförmiges Oberteil 2, ein plattenförmiges Unterteil 3 und einen weitgehend plattenförmig ausgebildeten Gelenkeinsatz 4.

Das Oberteil 2 ist an seiner Oberseite eben ausgebildet, so daß dadurch eine Stützfläche 5 entsteht, auf der verschiedenartige Vorsprünge 6, 7 angeordnet sind, die der Verankerung des Oberteils 2 in einem Wirbelknochen dienen, der mit seiner einem Zwischenwirbelraum zugewandten Endfläche auf der Stützfläche 5 aufliegt.

Das Oberteil 2 hat einen im wesentlichen rechteckigen Querschnitt, wobei in dem dargestellten Ausführungsbeispiel eine Längskante 8 nach außen gebogen ist.

An den beiden Schmalseiten dieses Rechteckes ist die Dicke des plattenförmigen Oberteiles 2 kleiner als im zentralen Bereich, so daß sich längs der schmalen Seiten des Oberteils 2 jeweils parallel zu diesen Kanten verlaufende, nach unten weisende Rücksprünge 9 ausbilden, die zur Außenseite hin offen sind. Zwischen den beiden Rücksprüngen 9 befindet sich der zentrale Bereich des Oberteils 2, der somit eine größere Dicke oder Höhe aufweist und somit einen zwischen den beiden Rücksprüngen 9 ausgebildeten, nach unten weisenden Vorsprung 10 ausbildet. Dieser wird durch eine im wesentlichen parallel zur Stützfläche 5 verlaufende Unterseite 11 begrenzt, in der sich eine kugelige Vertiefung 12 befindet, diese bildet eine Lagerschale aus für den Gelenkeinsatz 4.

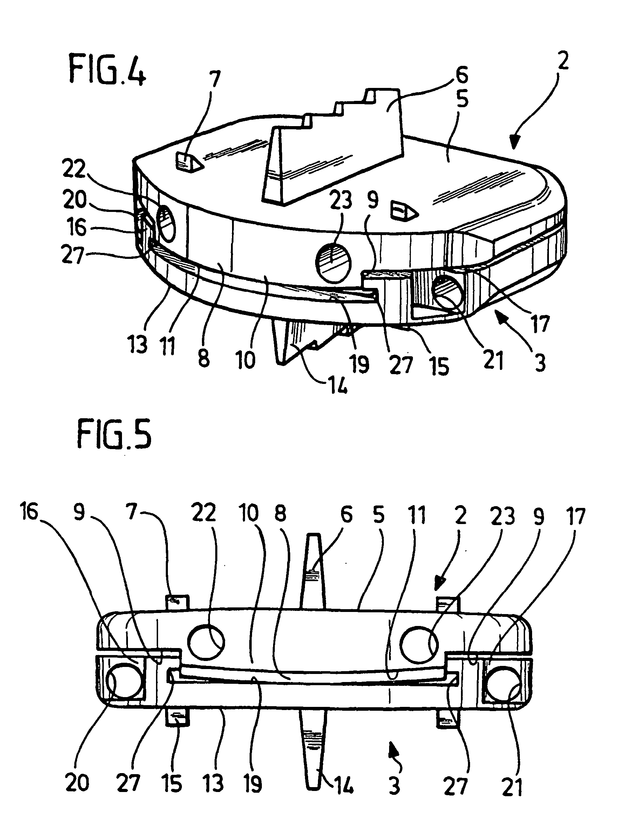

Das Unterteil 3 des Zwischenwirbelimplantates 1 ist ebenfalls plattenförmig ausgebildet und weist an seiner Unterseite eine ebene Stützfläche 13 mit Vorsprüngen 14 und 15 auf, die den Vorsprüngen 6 und 7 der Stützfläche 5 entsprechen. Auf der der Stützfläche 13 abgewandten Seite ist die Dicke des Unterteils 3 im zentralen Bereich geringer als in einem außenliegenden Bereich. Dieser außenliegende Bereich mit größerer Dicke hat die Form eines U mit zwei parallelen Schenkeln 16, 17, die parallel zu den Schmalkanten des im Querschnitt ähnlich wie das Oberteil 2 ausgebildeten Unterteiles 3 verlaufen, und mit einem die beiden Schenkel 16 und 17 an einer Seite verbindenden Steg 18. Der von den Schenkeln 16 und 17 und dem Steg 18 eingeschlossene Bereich bildet eine zentrale Vertiefung 19, deren Fläche im wesentlichen der Fläche des zentralen Vorsprunges 10 des Oberteils 2 entspricht, während die Anordnung und die Erstreckung der Schenkel 16 und 17 im wesentlichen der Anordnung und Erstreckung der Rücksprünge 9 am Oberteil 2 entsprechen. Es ist dadurch möglich, Oberteil 2 und Unterteil 3 so aufeinanderzulegen, daß der zentrale Vorsprung 10 des Oberteils 2 in die zentrale Vertiefung 19 eintaucht, während die Schenkel 16 und 17 des Unterteils 3 in die Rücksprünge 9 des Oberteils 2 eintauchen (Figur 4), in dieser Stellung sind Oberteil 2 und Unterteil 3 maximal einander angenähert und weisen eine minimale Bauhöhe auf.

Die Abmessungen sind dabei so gewählt, daß im wesentlichen die jeweiligen Rücksprünge durch die eintauchenden Vorsprünge vollständig ausgefüllt werden.

In die beiden Schenkel 16 und 17 des Unterteils 3 sind von deren freien Enden her parallel zu diesen Schenkeln 16, 17 verlaufend Sacklochbohrungen 20 und 21 eingearbeitet, deren Durchmesser im Verhältnis zur Höhe der Schenkel 16, 17 relativ groß ist, dieser Durchmesser ist tatsächlich größer als die Dicke oder Höhe des Unterteils 3 im Bereich der zentralen Vertiefung 19.

In den zentralen Vorsprung 10 des Oberteils 2 sind in der Nähe von dessen Seitenkanten Sacklochbohrungen 22 und 23 eingearbeitet, die parallel zu den Sacklochbohrungen 20 und 21 im Unterteil 3 verlaufen. Auch diese Sacklochbohrungen 22 und 23 haben einen relativ großen Durchmesser, der einem wesentlichen Teil der Höhe des Vorsprunges 10 entspricht und gröBer ist als die Dicke des Oberteils 2 im Bereich der Rücksprünge 9.

Wenn Oberteil 2 und Unterteil 3 in der beschriebenen Weise dicht aneinander anliegen, überlappen sich die Sacklochbohrungen 20 und 21 des Unterteils 3 und die Sacklochbohrungen 22 und 23 des Oberteils 2 in Richtung der Höhe des Zwischenwirbelimplantates 1 zumindest teilweise, wie dies aus der Darstellung der Figuren 4 und 5 deutlich wird.

Die Sacklochbohrungen 20, 21, 22 und 23 dienen als Aufnahmen für stiftförmige Verlängerungen eines in der Zeichnung nicht dargestellten Handhabungsinstrumentes und bilden somit Angriffselemente für dieses Handhabungsinstrument, welches auf diese Weise getrennt am Oberteil 2 und am Unterteil 3 angreift. Es ist mit diesem Handhabungsinstrument möglich, Oberteil 2 und Unterteil 3 des Zwischenwirbelimplantates 1 in einen Zwischenwirbelraum einzuführen, dabei erleichtert die sehr geringe Bauhöhe des Zwischenwirbelimplantates 1 dieses Einführen, das im wesentlichen ohne große Aufweitung des Zwischenwirbelraumes möglich ist.

Nach dem Einführen des Oberteils 2 und des Unterteils 3 in diese Weise können die beiden Teile des Zwischenwirbelimplantates 1 aufgespreizt werden, d.h. ihr Abstand wird beispielsweise mit Hilfe des das Oberteil 2 und das Unterteil 3 haltenden Handhabungsinstrumentes vergrößert.

In dieser aufgespreizten Lage von Oberteil 2 und Unterteil 3 ist es möglich, den Gelenkeinsatz 4 zwischen Oberteil 2 und Unterteil 3 einzuschieben.

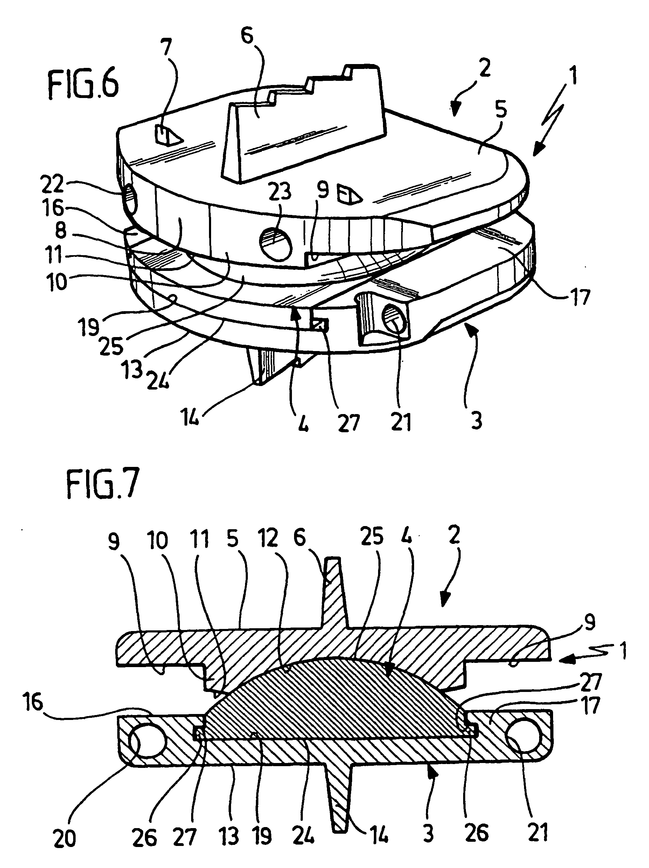

Dieser Gelenkeinsatz 4 ist im wesentlichen in Form einer Platte aufgebaut, die eine ebene Unterseite 24 und eine kugelig aufgewölbte Oberseite 25 aufweist. Die Außenabmessungen des plattenförmigen Gelenkeinsatzes 4 entsprechen denen der zentralen Vertiefung 19 im Unterteil 3, so daß der Gelenkeinsatz 4 diese Vertiefung ausfüllend in diese eingeschoben werden kann, und zwar von der Seite her, auf die sich die Sacklochbohrungen 20, 21, 22, 23 öffnen. Dabei greifen Führungsleisten 26 an den Seitenkanten des Gelenkeinsatzes 4 in entsprechende Führungsnuten 27 in den Schenkeln 16, 17 ein, so daß eine Einschubführung für den Gelenkeinsatz 4 gebildet wird, die diesen nach dem Einsetzen im Unterteil 3 festlegen. Der eingeschobene Gelenkeinsatz 4 füllt nach dem Einschieben die Vertiefung 19 aus und steht mit seiner kugelig gewölbten Oberseite 25 nach oben über die Oberseite des Unterteiles 3 hervor, die kugelige Oberseite 25 taucht dabei komplementär in die kugelig gewölbte Vertiefung 12 an der Unterseite des Vorsprunges 10 ein und bildet dort mit Oberteil 2 ein Kugelgelenk aus, welches eine gewisse Verschwenkbarkeit des Oberteils 2 gegenüber dem Unterteil 3 ermöglicht (Figur 7).

Der Gelenkeinsatz 4 kann an seiner ebenen Unterseite 24 einen Rastvorsprung 28 tragen, der beim Einschieben des Gelenkeinsatzes 4 in das Unterteil 3 elastisch in eine Rastausnehmung 29 einrastet, die sich am Boden der Vertiefung 19 befindet; dadurch wird der Gelenkeinsatz 4 auch in Einschubrichtung in der Vertiefung 19 festgelegt.

Oberteil 2 und Unterteil 3 sind vorzugsweise aus körperverträglichem Metall hergestellt, beispielsweise aus Titan, während der Gelenkeinsatz 4 vorzugsweise aus einem ebenfalls körperverträglichen Kunststoffmaterial besteht, beispielsweise aus Polyethylen. Diese Stützflächen 5 bzw. 13 können besonders knochenverträglich ausgebildet sein, beispielsweise kann diese Fläche durch eine Beschichtung aufgerauht werden, so daß sich eine optimale Verankerung mit dem benachbarten Knochenmaterial ergibt.

## Patentansprüche

1. Zwischenwirbelimplantat mit einem ersten Teil (2), das eine äußere Stützfläche (5) für einen ersten Wirbelkörper und eine innere Fläche mit wenigstens einem nach innen ragenden Vorsprung (10) aufweist, und einem zweiten Teil (3) mit einer äußeren Stützfläche (13) für einen dem ersten Wirbelkörper benachbarten zweiten Wirbelkörper und einer inneren Fläche, welche als eine Vertiefung (19) ausgebildet ist, die als Aufnahme für einen Einsatz (4) ausgestaltet ist, der bei der späteren Verwendung mit dem ersten Teil (2) zusammenwirkt, **dadurch gekennzeichnet, daß** der Vorsprung (10) des ersten Teils (2) in der Vertiefung (19) des zweiten Teils verschachtelbar ist, um die beiden Teile (2, 3) ohne den Einsatz (4) gemeinsam in einen Zwischenraum zwischen den Wirbelkörpern einzuführen.

2. Zwischenwirbelimplantat nach Anspruch 1, **dadurch gekennzeichnet, daß** es über Angriffselemente (20, 21, 22, 23) für ein Handhabungsinstrument verfügt, mit Hilfe dessen das erste (2) und das zweite Teil (3) in den Zwischenraum einführbar sind.

3. Zwischenwirbelimplantat nach Anspruch 2, **dadurch gekennzeichnet, daß** die Angriffselemente (20, 21, 22, 23) Einstecköffnungen für stiftförmige Halteelemente eines Handhabungsinstrumentes sind.

4. Zwischenwirbelimplantat nach Anspruch 3, **dadurch gekennzeichnet, daß** sich die Einstecköffnungen (20, 21, 22, 23) im wesentlichen parallel zu den Stützflächen (5, 13) erstrecken.

5. Zwischenwirbelimplantat nach einem der vorhergehenden Ansprüche, **dadurch gekennzeichnet, daß** das zweite Teil (3) eine seiner zugeordneten Stützfläche (13) gegenüberliegende zentrale Vertiefung (19) aufweist, die von einem U-förmigen Rand (16, 17, 18) umgeben ist.

6. Zwischenwirbelimplantat nach Anspruch 5, **dadurch gekennzeichnet, daß** das erste Teil (2) einen im wesentlichen komplementär in die Vertiefung (19) passenden zentralen Vorsprung (10) trägt.

7. Zwischenwirbelimplantat nach Anspruch 5 oder 6, **dadurch gekennzeichnet, daß** die Angriffselemente (20, 21) des zweiten Teils (3) an den beiden Enden des U-förmigen Randes (16, 17, 18) angeordnet sind.

8. Zwischenwirbelimplantat nach Anspruch 6 oder 7, **dadurch gekennzeichnet, daß** die Angriffselemente (22, 23) des ersten Teils (2) an dem zentralen Vorsprung (10) des ersten Teils (2) angeordnet sind.

9. Zwischenwirbelimplantat nach Anspruch 8, **dadurch gekennzeichnet, daß** die Angriffselemente (22, 23) des ersten Teils (2) nahe der seitlichen Ränder des zentralen Vorsprungs (10) angeordnet sind.

10. Zwischenwirbelimplantat nach einem der vorhergehenden Ansprüche, **dadurch gekennzeichnet, daß** das erste Teil (2) und/oder das zweite Teil (3) im wesentlichen plattenförmig ausgebildet sind.

11. Zwischenwirbelimplantat nach einem der vorhergehenden Ansprüche, **dadurch gekennzeichnet, daß** es einen als Gelenkeinsatz (4) ausgebildeten Einsatz aufweist, der in die als Aufnahme ausgebildete Vertiefung (19) des zweiten Teils (2) einfügbar ist.

12. Zwischenwirbelimplantat nach Anspruch 11, **dadurch gekennzeichnet, daß** der Gelenkeinsatz (4) eine kugelige Oberseite (25) aufweist, die in eine entsprechend kugelig gewölbte Vertiefung (12) des ersten Teils (2) eingreift.

13. Zwischenwirbelimplantat nach Anspruch 12, **dadurch gekennzeichnet, daß** die kugelige Vertiefung (12) in dem zentralen Vorsprung (10) des ersten Teils (2) angeordnet ist.

14. Zwischenwirbelimplantat nach einem der Ansprüche 11 bis 13, **dadurch gekennzeichnet, daß** der Gelenkeinsatz (4) von der Seite in die Aufnahme (19) einschiebbar ist, die die Angriffselemente (20, 21, 22, 23) für ein Handhabungsinstrument trägt.

15. Zwischenwirbelimplantat nach Anspruch 14, **dadurch gekennzeichnet, daß** der Gelenkeinsatz (4) längs einer Führung (26, 27) in die Aufnahme (19) einschiebbar ist.

16. Zwischenwirbelimplantat nach einem der Ansprüche 11 bis 15, **dadurch gekennzeichnet, daß** der Gelenkeinsatz (4) im wesentlichen plattenförmig ausgebildet ist.

17. Zwiechenwirbelimplantat nach einem der Ansprüche 12 bis 16, **dadurch gekennzeichnet, daß** der Gelenkeinsatz (4) die zentrale Aufnahme (19) im wesentlichen vollständig ausfüllt und mit der kugeligen Oberseite (25) aus der Aufnahme (19) hervorsteht.

18. Zwischewirbelimplantat nach einem der vorhergehenden Ansprüche, **dadurch gekennzeichnet, daß** in der verschachtelten Anordnung die Gesamthöhe des mit dem ersten Teil (2) verschachtelten zweiten Teils (3) kleiner ist als die Summe der getrennt betrachteten Höhen des ersten Teils (2) und des zweiten Teils (3).

19. Zwischenwirbelimplantat nach einem der Ansprüche 2 bis 18, **dadurch gekennzeichnet, daß** wenigstens in dem Vorsprung (10) des ersten Teils (2) und sich gegenüberliegenden Seitenwandungen (16, 17) des zweiten Teils (3) Angriffselemente (20, 21, 22, 23) für das Handhabungsinstrument vorgesehen sind, wobei im verschachtelten Zustand des ersten und des zweiten Teils (2, 3) die Angriffselemente (20, 21, 22, 23) nebeneinander liegen und sich in Richtung der Höhe des Zwischenwirbelimplantats (1) zumindest teilweise überlappen.

## Claims

1. An intervertebral implant comprising a first part (2) having an outer supporting surface (5) for a first vertebrae and having an inner surface having at least one inwardly extending protrusion (10), and a second part (3) having an outer supporting surface (13) for a second vertebrae adjacent to the first vertebrae and having an inner surface formed as a recess (19) which is constructed to receive an insert (4) which, in use, cooperates with the first part (2), **characterized by** the fact that the protrusion (10) of the first part (2) is nestable in the recess (19) of the second part (3) for inserting both parts (2, 3) together without the insert (4) into an intervertebral space.

2. An intervertebral implant according to claim 1 **characterized by** comprising engagement means (20, 21, 22, 23) for a handling instrument by means of which the first (2) and the second part (3) can be inserted into the intervertebral space.

3. An intervertebral implant according to claim 2, **characterized by** the fact that the engagement means (20, 21, 22, 23) are inserting apertures for pin-shaped engaging elements of the handling instrument.

4. An intervertebral implant according to claim 3, **characterized by** the fact that the inserting apertures (20, 21, 22, 23) extend substantially parallel to the supporting surfaces (5, 13).

5. An intervertebral implant according to one of the preceding claims, **characterized by** the fact that the second part (3) comprises a central recess opposite to its respective supporting surface (13) and surrounded by an U-shaped rim (16, 17, 18).

6. An intervertebral implant according to claim 5, **characterized by** the fact that the first part (2) carries a central protrusion (10) complementary fitting into the recess (19).

7. An intervertebral implant according to claim 5 or 6, **characterized by** the fact that the engagement means (20, 21) of the second part (3) are located at both ends of the U-shaped rim (16, 17, 18).

8. An intervertebral implant according to claim 6 or 7, **characterized by** the fact that the engagement means (22, 23) of the first part (2) are located at the central protrusion (10) of the first part (2).

9. An intervertebral implant according to claim 8, **characterized by** the fact that the engagement means (22, 23) of the first part (2) are located close to the lateral edges of the central protrusion (10).

10. An intervertebral implant according to one of the preceding claims, **characterized by** the fact that the first part (2) and/or the second part (3) a formed substantially plate shaped.

11. An intervertebral implant according to one of the proceeding claims, **characterized by** having an insert being formed as a joint insert which can be inserted into the recess (19) of the second part (2) being formed as a receiver.

12. An intervertebral implant according to claim 11, **characterized by** the fact that the joint insert (4) has a spherical top (25) engaging a respectively spherical formed indentation (12) of the first part (2).

13. An intervertebral implant according to claim 12, **characterized by** the fact that the spherical indentation (12) is located in the central protrusion (10) of the first part (2).

14. An intervertebral implant according to one of the claims 11 to 13, **characterized by** the fact that the joint insert (4) can be pushed into the receiver 19 carrying the engagement means (20, 21, 22, 23) for the handling instrument.

15. An intervertebral implant according to claim 14 **characterized by** the fact that the joint insert (4) can be pushed along a guide (26, 27) into the receiver (19).

16. An intervertebral implant according to one of claims 11 to 15, **characterized by** the fact that the joint insert (4) is formed substantially plate-shaped.

17. An intervertebral implant according to on of claims 12 to 16, **characterized by** the fact that the joint insert (4) occupies the central receiver (19) substantially completely and protrudes with its spherical top (25) out of the receiver (19).

18. An intervertebral implant according to one of the preceding claims, **characterized by** the fact that in the nested condition the total height of the second part (3) nested with the first part (2) is less than the additive heights of the first part (2) and the second part (3) taken separately.

19. An intervertebral implant according to one of claims 2 to 18, **characterized by** engagement elements (20, 21, 22, 23) for the handling instrument being located at least in the protrusion (10) of the first part (2) and in the opposed side walls (16, 17) of the second part (3), wherein in the nested condition of the first and the second parts (2, 3) the engagement elements (20, 21, 22, 23) lie side by side and overlap at least in part in the direction of the height of the intervertebral implant (1).

## Revendications

1. Implant intervertébral constitué d'une première partie (2), qui présente une surface d'appui externe (5) destinée à un premier corps vertébral et une surface interne ayant au moins une saillie (10) pointant vers l'intérieur, et d'une seconde partie (3), ayant une surface d'appui externe (13) destinée à un second corps vertébral voisin du premier corps vertébral et une surface interne qui est élaborée sous la forme d'un évidemment (19), qui est conçu pour servir de logement à une pièce rapportée (4), qui fonctionne conjointement avec la première partie (2) dans le cadre d'une utilisation ultérieure, **caractérisé en ce que** la saillie (10) de la première partie (2) peut s'imbriquer dans l'évidement (19) de la seconde partie, en vue d'une introduction conjointe des deux parties (2, 3) sans la pièce rapportée (4) dans un espace intermédiaire situé entre les deux corps vertébraux.

2. Implant intervertébral selon la revendication 1, **caractérisé en ce qu'**il dispose d'éléments de prise (20, 21, 22, 23) prévus pour un instrument de manipulation, à l'aide duquel il est possible d'introduire la première (2) et la seconde partie (3) dans l'espace intermédiaire.

3. Implant intervertébral selon la revendication 2, **caractérisé en ce que** les éléments de prise (20, 21, 22, 23) sont des ouvertures d'insertion destinées à des éléments de retenue en forme de tige d'un instrument de manipulation.

4. Implant intervertébral selon la revendication 3, **caractérisé en ce que** les ouvertures d'insertion (20, 21, 22, 23) s'étendent sensiblement parallèlement aux surfaces d'appui (5, 13).

5. Implant intervertébral selon l'une quelconque des revendications précédentes, **caractérisé en ce que** la seconde partie (3) présente un évidement central (19) situé à l'opposé de sa surface d'appui correspondante (13), lequel évidement est entouré d'un bord en forme de U (16, 17, 18).

6. Implant intervertébral selon la revendication 5, **caractérisé en ce que** la première partie (2) présente une saillie centrale (10) s'adaptant de manière sensiblement complémentaire à l'évidement (19).

7. Implant intervertébral selon la revendication 5 ou 6, **caractérisé en ce que** les éléments de prise (20, 21) de la seconde partie (3) sont aménagés aux deux extrémités du bord en forme de U (16, 17, 18).

8. Implant intervertébral selon la revendication 6 ou 7, **caractérisé en ce que** les éléments de prise (22, 23) de la première partie (2) sont aménagés sur la saillie centrale (10) de la première partie (2).

9. Implant intervertébral selon la revendication 8, **caractérisé en ce que** les éléments de prise (22, 23) de la première partie (2) sont aménagés à proximité des bords latéraux de la saillie centrale (10).

10. Implant intervertébral selon l'une quelconque des revendications précédentes, **caractérisé en ce que** la première partie (2) et/ou la seconde partie (3) sont élaborées sensiblement en forme de plaques.

11. Implant intervertébral selon l'une quelconque des revendications précédentes, **caractérisé en ce qu'**il présente une pièce rapportée conformée en pièce d'articulation rapportée (4), qui peut être introduite dans l'évidement (19) conçu comme logement de la seconde partie (2).

12. Implant intervertébral selon la revendication 11, **caractérisé en ce que** la pièce d'articulation rapportée (4) présente une face supérieure sphérique (25) qui s'emboîte dans un évidement incurvé sous forme sphérique (12) correspondant de la première partie (2).

13. Implant intervertébral selon la revendication 12, **caractérisé en ce que** l'évidement sphérique (12) est aménagé dans la saillie centrale (10) de la première partie (2).

14. Implant intervertébral selon l'une quelconque des revendications 11 à 13, **caractérisé en ce que** la pièce d'articulation rapportée (4) peut être insérée de côté dans le logement (19), qui porte les éléments de prise (20, 21, 22, 23) prévus pour un instrument de manipulation.

15. Implant intervertébral selon la revendication 14, **caractérisé en ce que** la pièce d'articulation rapportée (4) peut être insérée le long d'une glissière (26, 27) dans le logement (19).

16. Implant intervertébral selon l'une quelconque des revendications 11 à 15, **caractérisé en ce que** la pièce d'articulation rapportée (4) est réalisée sensiblement sous la forme d'une plaque.

17. Implant intervertébral selon l'une quelconque des revendications 12 à 16, **caractérisé en ce que** la pièce d'articulation rapportée (4) remplit le logement central (19) sensiblement dans sa totalité, la face supérieure sphérique (25) dépassant du logement (19).

18. Implant intervertébral selon l'une quelconque des revendications précédentes, **caractérisé en ce que**, selon un aménagement à l'état imbriqué, la hauteur totale de la seconde partie (3) imbriquée avec la première partie (2) est inférieure à la somme des hauteurs, considérées séparément, de la première partie (2) et de la seconde partie (3).

19. Implant intervertébral selon l'une quelconque des revendications 2 à 18, **caractérisé en ce que** l'on prévoit des éléments de prise (20, 21, 22, 23) pour l'instrument de manipulation au moins dans la saillie (10) de la première partie (2) et dans des parois latérales (16, 17), situées de manière opposée, de la seconde partie (3), dans lequel, lorsque les première et seconde parties (2, 3) sont à l'état imbriqué, les éléments de prise (20, 21, 22, 23) se trouvent l'un à côté de l'autre et se chevauchent, au moins partiellement, dans le sens de la hauteur de l'implant intervertébral (1).
